(19) **Europäisches Patentamt**

**European Patent Office**

**Office européen des brevets**

(11) **EP 0 823 253 B1**

(12) **FASCICULE DE BREVET EUROPEEN**

(45) Date de publication et mention
de la délivrance du brevet:
**18.05.2005 Bulletin 2005/20**

(51) Int Cl.⁷: **A61K 7/15**

(21) Numéro de dépôt: **97480047.6**

(22) Date de dépôt: **05.08.1997**

(54) **Produit de rasage et d'après rasage**

Rasier- und After-Shavemittel

Shaving and after-shave product

(84) Etats contractants désignés:
**DE ES FR GB IT**

(30) Priorité: **06.08.1996 FR 9610088**

(43) Date de publication de la demande:
**11.02.1998 Bulletin 1998/07**

(73) Titulaire: **Roard, Dany Lilian**
**06250 Mougins (FR)**

(72) Inventeur: **Roard, Dany Lilian**
**06250 Mougins (FR)**

(56) Documents cités:
**WO-A-95/05153**     **DE-A- 2 419 945**
**US-A- 4 129 942**     **US-A- 5 449 512**

**Description**

La présente invention concerne une préparation qui remplit, à la fois, le rôle de produit de rasage et de produit d'après-rasage et ce, en une seule application. En effet, cette préparation, anhydre, non-moussante satisfait aux besoins de lubrification et de protection de l'épiderme lors du rasage à la main et le rasage terminé, le film laissé sur l'épiderme, grâce aux agents rentrant dans sa composition, exerce une action adoucissante, rafraîchissante, tonifiante, hydratante, anti-irritation dont l'effet persiste plusieurs heures après l'application. Outre sa double activité, l'intérêt de l'invention tient dans l'infime quantité de produit nécessaire pour effectuer le rasage (quelques gouttes). La préparation peut donc être logée dans des volumes inhabituellement petits, voire dans le manche d'un rasoir jetable sans modification de l'encombrement de celui-ci.

Que ce soit chez l'homme ou chez la femme, le rasage s'effectue soit à l'aide d'un rasoir mécanique en appliquant préalablement sur la peau mouillée un produit de rasage, soit à l'aide d'un rasoir électrique avec ou sans préparation préalable de la peau. Sur ces deux modes de rasage, nous nous sommes intéressés à l'utilisation du rasoir mécanique à une ou plusieurs lames coupantes, associé à un produit de rasage. Ce dernier assure une action lubrifiante rendue nécessaire du fait de la pression et de la traction exercée par le dispositif de rasage sur l'épiderme. En effet lorsque cette lubrification est insuffisante, l'épiderme forme un bourrelet et la lame coupante risque de le blesser, soit sous forme de coupure, soit en l'irritant (plaques rouges), ce qui rend nécessaire l'utilisation d'un après rasage efficace.

Pour les besoins de cette lubrification il existe nombre de produits de rasage, qui sont : le savon à barbe, la mousse à raser en bombe ou en gel, la crème à raser, le gel de rasage moussant et le gel de rasage non-moussant. C'est ce dernier type qui se rapproche le plus de la présente invention puisqu'elle est aussi non-moussante. Nous n'avons toutefois relevé aucun produit de rasage (inclus les gels de rasage non-moussants) qui exerce simultanément les capacités de produit de rasage et de produit d'après rasage.

Concernant la fonction de produit de rasage, les brevets de Alan John ROBERTSON (GB-B-2167429° et de Peter DUNBAR (GB-93/00573) revendiquent tous deux la formation d'un gel aqueux non-moussant. Alan John ROBERTSON part d'une solution hydro-alcoolique (5 à 50 % en alcool éthylique) de carboxyméthylène neutralisé (0.05 à 2 %), incluant les agents courants tels : menthol, anti-UV, agent solubilisant et parfum. Les capacités lubrifiantes étant assurées par le gel hydro-alcoolique de carboxypolyméthylène, ce gel s'applique directement sur l'épiderme avant le rasage.

Peter DUNBAR améliore les performances du précédent brevet, basé lui aussi, sur un gel hydro-alcoolique (2 à 52% de polyol, préférentiellement du Glycérol) de carboxyméthylène neutralisé (0,05 à 4 %).

Pour l'essentiel, Peter DUNBAR remplace donc l'alcool à courte chaîne carbonée par un polyol de préférence le glycérol qui adoucit et lubrifie mieux l'épiderme qu'un alcool volatil tout en évitant le dessèchement de l'épiderme et son irritation. Ces gels de rasage non-moussants, tout comme le produit de rasage et d'après rasage de la présente invention, ne nécessitent pas de rinçage, leurs points commun, outre les capacités de produit de rasage, se bornent à cela.

Concernant la fonction de produit d'après rasage, le brevet de M.S. SIMMONS (US 5 449 512 A) et son extension internationale WO 96/05901 présentent une lotion d'après rasage qui, selon l'usage, est parfumée et contient des agents de conditionnement de l'épiderme, dont l'originalité est :

- de ne contenir qu'une faible teneur en alcool (C1-C6) : 20 à 30% alors que les teneurs vont habituellement de 50 à 90 %,
- d'incorporer à la préparation 50 à 60% de méthyle siloxane volatile, viscosité de 0.6 à 1.0 est, qui se répartit très facilement sur la peau et rend la peau douce et lisse après séchage.
- Et enfin d'être anhydre.

Mais comme les autres brevets de lotion d'après rasage, il n'est fait aucune mention de double capacité de produit de rasage qui serait donnée à cette préparation. Pour ce qui est de la composition, le point commun avec la présente invention est d'être anhydre : en effet, la présence éventuelle de méthyle siloxane dans la présente invention, compte tenu de sa double fonction, nécessite une viscosité comprise entre 100 et 500 cst pour que la rémanence du silicone soit suffisante une fois le rasage effectué.

Concernant les systèmes de rasage et les réservoirs ou cartouches qu'ils peuvent éventuellement contenir, le brevet DE 24 19 945 de rasoir électrique humide décrit un rasoir électrique dans lequel un dispositif permet la délivrance d'une « eau de rasage » pendant que le rasage électrique s'opère, par « vaporisation » de cette eau sur l'épiderme, cette eau de rasage pouvant avoir des capacités de rasage ou d'après rasage. Aucune précision n'est donnée des caractéristiques et de la composition de l'eau de rasage, le brevet ne décrit que le dispositif lui-même. En outre ce brevet couvre un mode d'utilisation essentiellement différent de celui de la présente invention car il concerne le seul rasage électrique, ce que ne couvre pas la présente invention.

Enfin, le brevet US 4, 129,942 de Neja H. DENIZMAN, décrit un dispositif incorporé ou incorporable dans un manche de rasoir mécanique permettant de recevoir le produit de rasage dans un compartiment et le produit d'après rasage dans l'autre compartiment. Ce dispositif se trouve essentiellement différent de la présente invention qui, elle permet de recevoir dans un seul réservoir aménagé dans le manche ou dans une cartouche insérable dans le manche le produit permettant de satisfaire, en une application, aux fonctions de produit de rasage et de produit d'après rasage.

L'étude de la présente invention trouve son origine dans l'observation faite que le rasage mécanique s'effectue le plus souvent au moyen de bombes aérosols délivrant une mousse lubrifiante. Il était intéressant de pouvoir, le cas échéant, supprimer les problèmes de rejet de gaz vecteur, voire celui de leur inflammabilité, et de réduire de façon très considérable les problèmes de rejet d'emballages usées. Pour cela les travaux de la présente invention se sont effectués en direction d'une préparation anhydre, non-moussante, sachant que de très petites quantités de produit peuvent satisfaire à la lubrification si leur étalement est préalablement assuré. En plus de la formulation d'un produit de rasage, nous avons travaillé l'activité substantive de la préparation de manière à ce que, au delà de l'opération de rasage, s'exerce les effets de conditionnement de l'épiderme propre a l'action d'un produit d'après rasage.

En définitive, la présente invention, produit de rasage et d'après rasage, se distingue des produits de rasage et des produits d'après rasage par l'application en quantité infime pour un bon rasage et un bon après rasage, sa double fonction et les différences que cela implique dans sa formulation. En outre, concernant les produits de rasage, tels que les gels, les crèmes et les mousses aérosols, la présente invention se distingue par son caractère anhydre.

La présente invention, préparation anhydre remplissant simultanément les fonctions de produit de rasage et de produit d'après rasage en assurant la lubrification et la protection de l'épiderme pendant le rasage et exerçant une action adoucissante, rafraîchissante, tonifiante, assainissante, odoriférante et antidéshydratation sur l'épiderme une fois le rasage terminé, est caractérisée par sa formulation à double effet, l'un immédiat pour satisfaire au rasage, est assuré par :

| des agents émollients, | 1.0 à 30% en poids, |
| des agents lubrifiants, | 1.0 à 90% en poids, |
| des agents odoriférants, | 1.0 à 30% en poids, |

l'autre substantif avec l'épiderme, permet d'apporter par delà l'opération de rasage l'effet de conditionnement d'un produit d'après rasage, est assuré par :

| des agents adoucissant-surgraissants | 0,5 à 30% en poids |
| des agents rafraîchissants | 0,5 à 5% en poids |
| des agents tonifiants | 0,5 à 5% en poids |
| des agents astringents | 0,5 à 5% en poids |
| des agents anti-irritation | 0,5 à 5% en poids |
| des agents assainissants | 0,5 à 5% en poids |
| des agents anti-déhydratation | 1,0 à 30% en poids |
| des agents odoriférants | 1,0 à 30% en poids |

La préparation est caractérisée par le fait qu'elle contient de préférence, d'une part, pour satisfaire à la fonction de produit de rasage :

❖ des agents émollients pris parmi :

- les alkyls phénols polyéthoxylés : 0.5 à 25.0 % en poids
- les polyéthylène glycols : 1.0 à 42.0 % en poids

❖ des agents lubrifiants pris parmi :

- les polyols esters d'acide gras : 3.0 à 47.0 % en poids
- les alkyls stéarates : 0.5 à 15.0 % en poids

❖ des agents odoriférants pris parmi :

- ceux d'un mélange d'huiles essentielles : 1.0 à 30.0 % en poids

d'autre part, pour satisfaire à la fonction de produit d'après rasage :

❖ des agents adoucissant-surgraissants pris parmi :

- les alcanolamides de coprah : 0.5 à 30 % en poids,

❖ des agents rafraîchissants pris parmi :

- le menthol : 0.1 à 5.0 % en poids
- ceux d'un mélange d'huiles essentielles 1.0 à 30 % en poids

❖ des agents tonifiants pris parmis :

- ceux d'un mélange d'huiles essentielles : 1.0 à 30 % en poids

❖ des agents astringents pris parmi :

- ceux d'un mélange d'huiles essentielles : 1.0 à 30 % en poids

❖ des agents anti-irritation pris parmi :

- le menthol : 0,1 à 5.0 % en poids

❖ des agents assainissants pris parmi :

- ceux d'un mélange d'huiles essentielles : 1.0 à 30 % en poids

❖ des agents anti-déshydratation pris parmi :

- l'isononanoate de cétéaryle : 1.0 à 30 % en poids

❖ des agents odoriférants pris parmi :

- ceux d'un mélange d'huiles essentielles : 1.0 à 30 % en poids.

La préparation est caractérisée par le fait qu'elle contient de plus :

jusqu'à 0,05 % en poids, de colorant alimentaire
jusqu'à 0.5 % en poids, d'agent anti UV
jusqu'à 0.3 % en poids, d'agent anti oxydant.

La présente invention est caractérisée en ce qu'elle contient de plus, 1.0 à 40.0 % en poids de méthyle siloxane qui peut éventuellement se substituer en tout ou en partie aux esters gras, cette huile de méthyle siloxane ayant une viscosité comprise entre 100 et 500 cst.
La présente invention est caractérisée par le fait qu'elle contient de plus, 1 à 40 % en poids d'huile de paraffine qui peut éventuellement se substituer en partie aux esters gras.
La présente invention dit caractérisée en ce que le mélange d'huiles essentielles est préférentiellement constitué :

- par des huiles essentielles de lavande, de cyprès, d'eucalyptus, de menthe, de romarin retenues pour leurs effets après rasage : en particulier pour leurs propriétés assainissantes, tonifiantes, rafraîchissantes, astringentes
- par des huiles essentielles de bois de rose, de coriandre, de mandarine, de vétyvert et de pamplemousse retenues pour leurs capacités odoriférantes .

Les effets lubrifiants sont ici assurés par les polyols esters d'acide gras, l'alcanolamide de coprah et l'alkyl stearate. Ils peuvent être remplacés, en partie par un dérivé siliconé, par exemple un méthyle siloxane éventuellement ramifié, ils peuvent être remplacés en partie par une huile de paraffine. L'agent anti-irritation utilisé dans cette invention sera préférentiellement le menthol, bien connu et beaucoup utilisé car il apporte en plus, un effet rafraîchissant.
La préparation produit de rasage et après rasage, objet de la présente invention, peut aussi recevoir un agent anti-oxydant pour garantir la conservation du mélange d'huiles essentielles. Cet agent de préférence sera le Buthyl Hydroxy Toluène.

Dans le cas de conditionnement en emballages transparents, il peut être souhaitable d'incorporer un agent anti UV, ce pourra être de préférence le Benzophénone.

La présente invention est caractérisée par le fait qu'elle est anhydre, c'est à dire qu'elle contient moins de 2% en poids d'eau et qu'elle est faite pour être appliquée sur un épiderme préalablement mouillé.

Le produit de rasage et d'après rasage de la présente invention est caractérisé par sa bonne compatibilité avec les emballages en aluminium, en verre, en polyéthylène, en P.V.C.

La présente invention, le produit de rasage et d'après rasage est non moussante et ne nécessite pas de rinçage, ce qui la caractérise.

La présente invention, ayant la double fonction de produit de rasage et d'après rasage, peut-être utilisé en tant que seul produit de rasage et d'après rasage, ce qui la caractérise. Pour cela, sur l'épiderme préalablement mouillé, il suffit de répartir quelques gouttes (150 à 250 mg).

La présente invention, fluide de rasage et d'après rasage, assure le nettoyage instantané du dispositif de rasage (une à deux secondes sous un filet d'eau) et prolonge ainsi sa durée d'utilisation, ce qui la caractérise.

La présente invention, produit de rasage et d'après rasage, est caractérisée en ce qu'elle requiert pour son utilisation d'infimes quantités de produit (150 à 250 mg pour le rasage d'une barbe, 800 à 1000 mg pour le rasage des jambes).

La préparation, objet de la présente invention est caractérisée en ce qu'elle est contenue dans la parti évidée d'un manche de rasoir jetable ordinaire, sans modification de l'encombrement extérieur du manche, lui donnant ainsi une autonomie allant jusqu'à 20 rasages de barbes et après-rasages.

La préparation, objet de la présente invention, est caractérisée en ce qu'elle est contenue dans une cartouche logeable dans le manche d'un type courant de rasoir à dispositif de rasage interchangeable sans modification de l'encombrement extérieur du manche, lui donnant ainsi une autonomie allant jusqu'à 20 rasages de barbes et après-rasages.

La petitesse de l'emballage servant à son conditionnement est caractéristique de cette invention.

Les exemples suivants, représentatifs de la composition du produit de rasage et d'après rasage, objet de la présente invention, sont donnés ci-après pour illustrer la procédure de fabrication. Ces exemples ne doivent pas être considérés comme limitatifs.

Exemple 1

| PRODUIT DE RASAGE ET D'APRES RASAGE 25076 | |
|---|---|
| DESIGNATION CTFA | % poids |
| mélange d'huiles essentielles* | 11.3 |
| glycéryl cocoate | 18.0 |
| PEG-7 | 16.2 |
| octyl stéarate | 11.7 |
| cocamide DEA | 17.1 |
| octyl phenol (oet)10 | 3.4 |
| isononanoate de cétéaryle | 21.2 |
| menthol cristallisé codex | 0.2 |
| colorant bleu alim.hydosoluble (sol 10%) | 0.5 |
| butyl hydroxy toluene | 0.1 |
| | 100.0 |

mise en oeuvre :
(*) Préparer le mélange d'huiles essentielles :
   lavande 43%, bois de rose 5%, menthe 5%, eucalyptus 20%, coriandre 7%, cyprès 20%

Y incorporer le menthol cristallisé codex, laisser sous agitation. La dissolution opérée, ajouter successivement le glycéryl cocoate, le PEG, l'octyl stéarate, l'isononanoate de cétéaryl, l'octyl phénol (oet) 10, le butyl hydroxy toluène.

Ajouter, sous agitation, le cocoamide DEA préalabement fondu.

Enfin, ajouter le colorant sous agitation vive.

Exemple 2

| PRODUIT DE RASAGE ET D'APRES RASAGE 2086 | |
| --- | --- |
| DESIGNATION CTFA | % poids |
| mélange d'huiles essentielles* | 20.0 |
| glycéryl cocoate | 30.0 |
| PEG-7 | 27.1 |
| octyl stéarate | 0.5 |
| cocamide DEA | 8.0 |
| isononanoate de cétéaryle | 13.0 |
| menthol cristallisé codex | 1.1 |
| benzophenone | 0.1 |
| butyl hydroxy Toluene | 0.2 |
| | 100.0 |

mise en oeuvre :
(*) Préparer le mélange d'huiles essentielles :
   lavande 27.0%, menthe 10.8%, eucalyptus 10.8%, pamplemousse 13.5%, vetyvert 8.1%, romarin 20.3%.

comme dans l'exemple précédent, le menthol cristallisé est ajouté au mélange d'huiles essentielles et mis sous agitation jusqu'à complète dissolution.

Ajouter alors successivement le glycéryl cocoate, le PEG, l'octyl stéarate, l'isononnanoate de cetearyle, l'octyl phénol (OET)10, le BHT, le benzophénone

Sous agitation, ajouter le cocamide DEA préalablement fondu.

Les exemples précédents sont donnés au titre d'illustration uniquement et ne constituent en rien une limitation aux différentes compositions que peut prendre la présente invention, laquelle peut avoir beaucoup d'autres formulations possibles en accord avec la composition donnée.

**Revendications**

1. Préparation anhydre, remplissant simultanément les fonctions de produit de rasage et de produit d'après rasage qui consiste en :

| des agents émollients | 1.0 à 30.0 % en poids |
| --- | --- |
| des agents lubrifiants | 1.0 à 90.0 % en poids |
| des agents odoriférants | 1.0 à 30.0 % en poids |
| agents des adoucissant-surgraissants | 0.5 à 30.0 % en poids |
| des agents rafraîchissants | 0.5 à 5 % en poids |
| des agents tonifiants | 0.5 à 5% en poids |
| des agents astringents | 0.5 à 5% en poids |
| des agents anti-irritation | 0.5 à 5% en poids |
| des agents assainissants | 0.5 à 5% en poids |
| des agents anti-déhydratation | 1.0 à 30% en poids |
| des agents odoriférants | 1.0 à 30% en poids |

❖ des agents émollients pris parmi

- les alkyls phénols polyéthoxylés :     0.5 à 25% en poids
- les polyéthylène glycols :     1.0 à 42.0 % en poids

❖ des agents lubrifiants pris parmi :

- les polyols esters d'acide gras : 3.0 à 47.0 % en poids
- les alkyls stéarates : 0.5 à 15.0 % en poids

❖ des agents odoriférants pris parmi :

- ceux d'un mélange d'huiles essentielles : 1.0 à 30.0 % en poids

❖ des agents adoucissant-surgraissants pris parmi :

- les alcanolamides de coprah : 0.5 à 30 % en poids,

❖ des agents rafraîchissants pris parmi :

- le menthol : 0.1 à 5.0 % en poids
- ceux d'un mélange d'huiles essentielles 1.0 à 30 % en poids

❖ des agents tonifiants pris parmis :

- ceux d'un mélange d'huiles essentielles : 1.0 à 30 % en poids

❖ des agents astringents pris parmi :

- ceux d'un mélange d'huiles essentielles : 1.0 à 30 % en poids

❖ des agents anti-irritation pris parmi :

- le menthol : 0.1 à 5.0 % en poids

❖ des agents assainissants pris parmi :

- ceux d'un mélange d'huiles essentielles : 1.0 à 30 % en poids

❖ des agents anti-déshydratation pris parmi :

- l'isononanoate de cétéaryle : 1.0 à 30 % en poids

❖ des agents odoriférants pris parmi :

- ceux d'un mélange d'huiles essentielles : 1.0 à 30 % en poids

**2.** Préparation , selon la revendication 1, **caractérisée par le fait qu'**elle est anhydre, c'est à dire qu'elle contient moins de 2% en poids d'eau et qu'elle est faite pour être appliquée sur un épiderme préalablement mouillé.

**3.** Préparation, selon l'une des revendications 1 à 2, **caractérisée en ce qu'**elle est non moussante et ne nécessite pas de rinçage.

**4.** Préparation, selon l'une des revendications 1 à 3, **caractérisée en ce qu'**elle est contenue dans la partie évidée d'un manche de rasoir jetable ordinaire, sans modification de l'encombrement extérieur du manche, lui donnant ainsi une autonomie allant jusqu'à 20 rasages de barbes et après-rasages.

**5.** Préparation, selon l'une des revendications 1 à 4, **caractérisée en ce qu'**elle est contenue dans une cartouche logeable dans le manche d'un type courant de rasoir à dispositif de rasage interchangeable sans modification de l'encombrement extérieur du manche, lui donnant ainsi une autonomie allant jusqu'à 20 rasages de barbes et après-rasages

**6.** Préparation, selon l'une des revendications 1 à 5, **caractérisée en ce qu'**elle contient de plus :

jusqu'à 0.05 % en poids, de colorant alimentaire

jusqu'à 0.5 % en poids, d'agent anti UV

jusqu'à 0.3 % en poids, d'agent anti oxydant.

7. Préparation, selon l'une des revendications 1 à 6, **caractérisée en ce qu'**elle contient de plus, 1.0 à 40.0 % en poids de méthyle siloxane qui peut éventuellement se substituer en tout ou en partie aux esters gras, cette huile de méthyle siloxane ayant une viscosité comprise entre 100 et 500 est.

8. Préparation, selon l'une des revendications 1 à 7, **caractérisée par le fait qu'**elle contient de plus, 1 à 40 % en poids d'huile de paraffine qui peut éventuellement se substituer en partie aux esters gras.

9. Préparation, selon l'une des revendications 1 à 8, **caractérisée en ce que** le mélange d'huiles essentielles est préférentiellement constitué :

- par des huiles essentielles de lavande, de cyprès, d'eucalyptus, de menthe, de romarin retenues pour leurs effets après rasage, en particulier pour leurs propriétés assainissantes, tonifiantes, rafraîchissantes, astringentes
- par des huiles essentielles de bois de rose, de coriandre, de mandarine, de vétyvert pamplernousse retenues pour leurs capacités odoriférantes.

**Patentansprüche**

1. Nicht wässriges Präparat, das zugleich die Funktion eines Rasiermittels und eines After-Shave-Produktes erfüllt und aus folgenden Zusätzen besteht :

| | |
|---|---|
| Emollientien | 1.0 bis 30.0% Gewicht |
| Gleitmittel | 1.0 bis 90.0% Gewicht |
| Duftstoffe | 1.0 bis 30.0% Gewicht |

| | |
|---|---|
| weichmachende, überfettende Wirkstoffe | 0.5 bis 30.0% Gewicht |
| erfrischende Wirkstoffe | 0.5 bis 5 % Gewicht |
| belebende Wirkstoffe | 0.5 bis 5 % Gewicht |
| adstringierende Wirkstoffe | 0.5 bis 5 % Gewicht |
| irritationshemmende Wirkstoffe | 0.5 bis 5 % Gewicht |
| desinfizierende Wirkstoffe | 0.5 bis 5 % Gewicht |
| Austrocknung verhindernde Wirkstoffe | 1.0 bis 30.0% Gewicht |
| Duftstoffe | 1.0 bis 30.0% Gewicht |

❖ Fanollientien, ausgewählt unter den :

- Phenol Polyethoxy Alkyls        0.5 bis 25.0 % Gewicht
- Polyethylene Glycols        1.0 bis 42.0 % Gewicht

❖ Gleitmittel, ausgewählt unter den :

- Fettsaureesterpolyolen        3.0 bis 47.0 % Gewicht
- Alkyl Stearate        0.5 bis 15.0 % Gewicht

❖ Duftstoffe, ausgewählt unter jenen :

- einer Mischung essentieller Öle        1.0bis 30.0 % Gewicht

❖ weichmachende und überfettende Wirkstoffe, ausgewählt unter den :

- Kopra-Alkanolamiden        0.5 bis 30 % Gewicht

- ❖ erfrischende Wirkstoffe, ausgewählt unter :

  - dem Menthol    0.1 bis 5.0 % Gewicht
  - jenen einer Mischung essentieller Öle    1.0 bis 30 % Gewicht

- ❖ tonisierende Wirkstoffe, ausgewählt unter :

  - jenen einer Mischung essentieller Öle    1.0 bis 30 % Gewicht

- ❖ adstringierende Wirkstoffe, ausgewählt unter :

  - jenen einer Mischung essentieller Öle    1.0 bis 30 % Gewicht

- ❖ Irritationshemmende Wirkstoff e:

  - Menthol    0.1 bis 5.0 % Gewicht

- ❖ desinfizierende Wirkstoffe, ausgewählt unter :

  - jenen einer Mischung essentieller Öle    1.0 bis 30 % Gewicht

- ❖ Austrocknung bekämpfende Wirkstoffe, ausgewählt unter :

  - Cetearyl Isononanoate    1.0 bis 30 % Gewicht

- ❖ Duftstoffe, ausgewählt unter :

  - jenen einer Mischung essentieller Öle    1.0 bis 30 % Gewicht

2. Präparat, das Anspruch 1 zufolge dadurch charakterisiert wird, dass es nicht wässrig ist, das heisst, dass es weniger als 2 Gewichtprozent Wasser enthält und auf eine vorher nass gemachte Haut angewendet wird

3. Präparat, das einer der Ansprüche von 1 bis 2 zufolge dadurch charakterisiert wird, dass es nicht schäumend ist und kein Abspülen benötigt.

4. Präparat, das einer der Ansprüche von 1 bis 3 zufolge dadurch charakterisiert wird, dass es im hohlen Teil eines Rasierklingenhalters eines gewöhnlichen Wegwerfgerätes enthalten ist, ohne dessen Ausmasse zu verändern, und ihm somit eine Autonomie von bis zu 20 Barthaar-Rasuren und After-Shave-Pflege verleiht.

5. Präparat, das einer der Ansprüche von 1 bis 4 zufolge dadurch charakterisiert wird, dass es in einer Patrone enthalten ist, die in den Griff eines gewöhlichen Rasiergerätes mit auswechselbarer Klinge einsetzbar ist, ohne dessen Ausmasse zu verändern, und ihm somit eine Autonomie von bis zu 20 Barthaar-Rasuren und After-Shave-Pflege verleiht.

6. Präparat, das einer der Ansprüche von 1 bis 5 zufolge dadurch charakterisiert wird, dass es zudem folgende Zusätze enthält :

   bis zu 0.05% Gewicht natürlicher Farbstoff
   bis zu 0.5 % Gewicht Lichtschutz
   bis zu 0.3 % Gewicht Antioxidantien

7. Präparat, das einer der Ansprüche von 1 bis 6 zufolge dadurch charakterisiert wird, dass es zudem 1.0 bis 40.0 Gewichtprozent Methylsiloxan enthält, das eventuell vollständig oder teilweise die Fettester ersetzen kann, da dieses Methylsiloxan-Öl eine Viskosität zwischen 100 und 500 cst besitzt.

8. Präparat, das einer der Ansprüche von 1 bis 7 zufolge dadurch charakterisiert wird, dass es zudem 1 bis 40.0 Gewichtprozent Paraffinöl enthält, das teilweise die Fettester ersetzen kann.

9. Präparat, das einer der Ansprüche von 1 bis 8 zufolge dadurch charakterisiert wird, dass seine Mischung essentieller Öle in erster Linie aus den folgenden Komponenten zusammengesetzt ist :

- essentielle Öle des Lavendels, Zypresse, Eukalyptus, Minze, Rosmarin, alle für ihre Wirkung für nach der Rasur ausgewählt, insbesondere für ihre desinfizierenden, belebenden, erfrischenden, adstringierenden Eigenschaften
- essentielle Öle des Rosenholzes, Korianders, Mandarine, Vetiver, Pampelmuse, alle für ihre wohlriechenden Eigenschaften ausgewählt.

**Claims**

1. An anhydrous preparation, simultaneously fulfilling the functions of a shaving product and an aftershave, consisting of :

| | |
|---|---|
| emolliating agents | 1.0 to 30.0% by weight |
| lubricating agents | 1.0 to 90.0% by weight |
| odorising agents | 1.0 to 30.0% by weight |

| | |
|---|---|
| soothing-superfatting agents | 0.5 to 30.0% by weight |
| refreshing agents | 05 to 5% by weight |
| toning agents | 0.5 to 5% by weight |
| astringency agents | 0.5 to 5% by weight |
| anti-irritant agents | 0.5 to 5% by weight |
| cleansing agents | 0.5 to 5% by weight |
| anti-dehydration agents | 1.0 to 30% by weight |
| odorising agents | 1.0 to 30% by weight |

- emolliating agents selected among :

  - phenol polyethoxyl alkyls        0.5 to 25% by weight
  - polyethylene glycols        1.0 to42.0% by weight

- lubricating agents selected among:

  - polyols of fatty acid        3.0 to 47.0% by weight
  - stearate alkyls        0.5 to 15.0% by weight

- odorising agents selected among :

  - those of a blend of essential oils        1.0 to 30.0% by weight

- soothing-superfatting agents selected among :

  - alcanolamides of copra:        0.5 to 30% by weight

- refreshing agents selected among :

  - menthol        0.1 to 5.0% by weight
  - those of a blend of essential oils:        1.0 to 30% by weight

- toning agents selected among:

  - those of a blend of essential oils:        1.0 to 30% by weight

- astringency agents selected among :

  - those of a blend of essential oils :       1.0 to 30% by weight

- anti-irritation agents selected among :

  - menthol       0.1 to 5.0% by weight

- cleansing agents selected among :

  - those of a blend of essential oils:       1.0 to 30% by weight

- anti-dehydration agents selected among :

  - isononanoate of cetearyle :       1.0 to 30% by weight

- odorising agents selected among :

  - those of a blend of essential oils :       1.0 to 30.0% by weight

2. A preparation, as specified in Claim 1, **characterised by** the fact that it is anhydrous, that is to say that it contains less than 2% by weight of water and that it is intended for application on an epidermis dampened beforehand.

3. A preparation, as specified in Claims 1&2, **characterised by** the fact that it is non-lathering and does not require rinsing.

4. A preparation, as specified in Claims 1 to 3, **characterised by** the fact that it is contained in the hollow part of the handle of an ordinary disposable razor, without modification to the external dimensions of the handle, providing in this way, sufficient product for up to 20 shaving and aftershave operations.

5. A preparation, as specified in Claims 1 to 4, **characterised by** the fact that it is contained in a cartridge capable of being inserted in the handles of current types of non-disposable razors without change to the external dimensions of the handle, providing in this way sufficient product for up to 20 shaving and aftershave operations.

6. A preparation, as specified in Claims 1 to 5, **characterised by** the fact that it contains in addition:

   up to 0.05% by weight of food-grade colouring
   up to 0.5% by weight of anti-UV agent
   up to 0.03% by weight of anti-oxidising agent

7. A preparation, as specified in Claims 1 to 6, **characterised by** the fact that it contains in addition, 1.0 to 40.0% by weight of methyl siloxane, which may be replaced in whole or in part by fatty esters, this oil of methyl siloxane having a viscosity of between 100 and 500 est.

8. A preparation, as specified in Claims 1 to 7, **characterised by** the fact that it contains in addition, 1.0 to 40.0% by weight of oil of paraffin, which may be replaced in part by fatty esters.

9. A preparation, as specified in Claims 1 to 8, **characterised by** the fact that the blend of essential oils consists preferentially of :

   - essential oils of lavender, cypress, eucalyptus, mint, and rosemary chosen for their aftershave effects, in particular for their cleansing, toning, refreshing and astringent properties.
   - essential oils of rosewood, coriander, mandarin, vetyvert and grapefruit chosen for their odorising properties.